# EUROPEAN PATENT APPLICATION

(11) **EP 4 524 155 A1**
(43) Date of publication of application: **19.03.2025**
(21) Application number: 23802601.7
(22) Date of filing: 19.04.2023
(51) Int. Cl.: C07K 16/10, C12N 15/13, C12N 15/866, A61P 31/14

(54) **MONOCLONAL ANTIBODY A38 AGAINST RIFT VALLEY FEVER VIRUS AND USE**

(30) Priority: 11.05.2022 CN 202210510320
(71) Applicant: Academy of Military Medical Sciences, PLA, Beijing 100850 (CN)
(72) Inventor: CHEN, Wei, Beijing 100071 (CN); LI, Jianmin, Beijing 100071 (CN); HAO, Meng, Beijing 100071 (CN); YU, Changming, Beijing 100071 (CN); HOU, Lihua, Beijing 100071 (CN); BIAN, Ting, Beijing 100071 (CN); CHEN, Yi, Beijing 100071 (CN); FANG, Ting, Beijing 100071 (CN); LIU, Shuling, Beijing 100071 (CN)
(74) Representative: dompatent von Kreisler Selting Werner - Partnerschaft von Patent- und Rechtsanwälten mbB
(86) International application number: PCT/CN2023/089236
(87) International publication number: WO 2023/216826

(57) **Abstract**

Disclosed is a monoclonal antibody against the Rift Valley fever virus. The antibody is screened and obtained by means of flow cytometric sorting and single-cell PCR technology, and has a unique CDR region. Further disclosed is the use of the antibody in the preparation of a drug for treating Rift Valley fever. The monoclonal antibody against the Rift Valley fever virus has high efficiency and specific activity against the Rift Valley fever virus; and also has the characteristics of high expression, high degree of humanization and good stability, and is suitable for industrial production.

## Description

### TECHNICAL FIELD

The present disclosure discloses an antibody, which belongs to the fields of microbiology and immunology.

The present disclosure claims priority of CN202210510320.6, with a title of "Monoclonal Antibody A38 Against Rift Valley Fever Virus and Use", and filling date of May 11,2022.

### BACKGROUND TECHNOLOGY

Rift Valley Fever (RVF) is a zoonotic mosquito-borne infectious disease caused by RVF virus, named after its initial isolation from the East African Great Rift Valley, which is mainly prevalent in the African region, with imported cases reported in Asia. In 2016, its first imported case of RVF was identified in China, and the potential risk of cross-regional transmission of RVF also poses a challenge to biosecurity in China. The World Health Organization (WHO) released a list of the viruses in need of current investigation in 2015, 2016, and 2018, respectively, all of which included RVF virus. In July 1997, the 7th meeting of the ad hoc Expert Group of States Parties to Biological Weapons Convention has listed RVF, along with Ebola, Marburg, in the viral warfare agent that attacks people. The clinical symptoms of RVF include fever, headache, and muscle & joint pain, while multiple organ involvement found in severe cases, with high mortality. In September 2020, Mauritania experienced an outbreak of RVF, reporting 75 confirmed cases and 25 deaths, with a mortality of 33.3%. Currently, RVF has no effective treatment options, with symptomatic supportive treatment as the main choices. Compared with the other drugs, monoclonal antibodies have advantages of clear targets, specific effects, rapid onset of action, and less side effects, making them become the hotspot in the field of research on antiviral therapies. The development of specific monoclonal antibody is of great significance for the prevention and control of RVF virus-related epidemic, as well as bioterrorism prevention and anti-bioterrorism.

As of February 2021, there are 6 novel anti-infective monoclonal antibodies out of the 100 new antibodies approved by the FDA, including 2 monoclonal antibodies each against anthrax and Ebola, which indicates that anti-infective monoclonal antibodies occupy a place in this field. With the continuous progress of antibody development technology and global attention to public health emergencies, the number of approved new anti-infective antibodies is rapidly increasing. The COVID-19 pandemic has pushed the development of new anti-infective antibodies to a new height, especially in the situation of continuous emergence of mutants, attracting more and more attention. In the field of development of antibodies against Ebola and SARS-COV-2 virus, both Regeneron and VIR in the United States have anti-Ebola monoclonal antibodies approved to market, and anti- COVID-19 monoclonal antibodies obtaining EUA approval. However, there are no anti-infective monoclonal antibodies approved for launch in China.

### SUMMARY OF THE DISCLOSURE

### Technical Problem

At present, no RVF vaccines and neutralizing monoclonal antibodies have been approved for marketing domestically and globally. Phase II clinical trials of the attenuated vaccine MP-12 developed by the United States Army Medical Research Institute of Infectious Diseases has been completed. The development of neutralizing monoclonal antibodies against RVF is currently in the laboratory research stage, mainly targeting the surface glycoproteins Gn and Gc of RVF virus. Gn protein mainly mediates virus recognizing host cell receptors, while Gc protein primarily mediates the membrane fusion between viruses and host cells. In 2018, Thomas A. Bowden et al. from the University of Oxford, UK, obtained the rabbit monoclonal antibody targeting Gn protein with neutralizing activity through flow cytometry sorting, whose protective activity is verified in a mouse model. German scientists reported in 2020 that two mouse monoclonal antibodies Gn3 and Gn32 targeting Gn protein are obtained through screening. Gn3 alone in mouse protection test reached the protection rate of 58%, while the "cocktail" treatment with Gn3 and Gn32 achieved complete protection, indicating better therapeutic prospects of the combination therapy. Gao Fu, Yan Jinghua and their teams have firstly isolated the highly efficient monoclonal antibody that neutralizes RVFV infection in a patient having recovered from RVFV infection, which is effective in the treatment of RVFV infection in mouse models and is accordingly expected to become a candidate drug for treating RVFV infection.

The purpose of the present disclosure is to provide a monoclonal antibody with high neutralizing activity, and to further provide its applications in the preparation of therapeutic drugs for RVF.

### Solution to Technical problem

For the above purposes, the present disclosure firstly constructs an adenovirus vector candidate vaccine against RVF. A monoclonal antibody against RVF is obtained through immunizing rhesus monkeys, and screening with flow cytometry sorting - single cell PCR, and the amino acid sequences of CDR1, CDR2, and CDR3 regions in the heavy chain variable region is shown as the amino acid sequence of positions 27-35, 53-60, and 99-112 of SEQ ID NO:1 respectively; and the amino acid sequences of CDR1, CDR2, and CDR3 regions in the light chain variable region is shown as the amino acid sequence of positions 26-34, 52-58, and 97-106 of SEQ ID NO:5 respectively.

In an optional embodiment, the amino acid sequence of the heavy chain variable region of the antibody is shown as SEQ ID NO:1, and the amino acid sequence of the light chain variable region of the antibody is shown as SEQ ID NO:5. In the present disclosure, a specific antibody with the heavy and light chain variable regions is named as "A38".

In a more optional embodiment, the amino acid sequence of the heavy chain constant region of the antibody is shown as SEQ ID NO:3, and the amino acid sequence of the light chain constant region of the antibody is shown as SEQ ID NO:7 or SEQ ID NO:9, wherein, SEQ ID NO:7 is the sequence of κ light chain constant region, and SEQ ID NO:9 is the sequence of λ light chain constant region. The heavy and light chain constant regions of the antibody are human-derived.

Secondly, the present disclosure provides a polynucleotide encoding the heavy and light chains of the monoclonal antibody, and the sequence of the polynucleotide encoding the heavy chain variable region is shown as SEQ ID NO:2, and the sequence of the polynucleotide encoding the light chain variable region is shown as SEQ ID NO:6.

In an optional embodiment, the sequence of the polynucleotide encoding the heavy chain constant region of the antibody is shown as SEQ ID NO:4, and the sequence of the the polynucleotide encoding the light chain constant region is shown as SEQ ID NO:8 or SEQ ID NO:10.

Thirdly, the present disclosure provides a functional element expressing the polynucleotide encoding the heavy chain and light chain of the monoclonal antibody. the functional element can be a conventional expression vector.

In an optional embodiment, the functional element is a linear expression cassette.

Fourthly, the present disclosure provides a host cell containing the aforementioned linear expression cassette.

In an optional embodiment, the cell is an Expi 293F cell.

In another optional embodiment, the cell is a CHO-S cell.

Finally, the present disclosure provides a use of the aforementioned monoclonal antibody in the preparation of therapeutic drugs for RVF.

### Benefical Effects of the present Disclosure

The monoclonal antibody provided in the present disclosure shows good neutralization and protective effects against cells infected with RVF virus. Results of the present disclosure demonstrate that the antibody has broad application prospects in the preparation of therapeutic drugs for RVF. The monoclonal antibody disclosed in the present disclosure also has the following technical advantages: (1) high binding activity, with dissociation constant (KD) of the recombinant monoclonal antibody to Gn protein less than 1 nM; (2) High neutralizing activity, with IC₅₀ in a cell model less than 1 nM; In a lethal mouse model, 20 ug per mouse can provide complete protection. (3) Good stability, since the antibody genes come from the same cell in the rhesus monkey, which are naturally paired.

### BRIEF DESCRIPTION OF THE DRAWINGS

Figure 1 provides the titers of IgG antibodies against proteins Gn and Gc in rhesus monkeys at different time points after immunization;
Figure 2 is the sorting strategy for Gn and Gc-specific memory B cells;
Figure 3 depicts the nucleic acid electrophoresis of two-round nested PCR amplification products of heavy chain;
Figure 4 shows the nucleic acid electrophoresis of two-round nested PCR amplification products of light chain;
Figure 5 is the nucleic acid electrophoresis of amplification products of heavy and light chain variable regions;
Figure 6 provides the amplification of leader sequence and constant region -poly (A) tail;
Figure 7 depicts the nucleic acid electrophoresis of linear expression cassettes of heavy and light chains;
Figure 8 shows the detection of binding activity of antibody expressed by linear expression cassette transfecting;
Figure 9 is the nucleic acid electrophoresis of amplification products of full-length heavy and light chains;
Figure 10 is the nucleic acid electrophoresis of expression vector pCNDA3.4 after linearization by double enzyme digestion;
Figure 11 is the double enzyme digestion identification of the expression vector by nucleic acid electrophoresis;
Figure 12 provides SDS-PAGE of the monoclonal antibody purified by affinity chromatography;
Figure 13 describes the detection of binding activity between the monoclonal antibody and Gn protein;
Figure 14 depicts the test of affinity between the monoclonal antibody and Gn protein;
Figure 15 shows the IC50 assay of the monoclonal antibody in a cell model;
Figure 16 is the protective effect of monoclonal antibody on RVFV-infected mice;
Figure 17 provides the results of weight changes in RVFV-infected mice treated with the monoclonal antibody;
Figure 18 describes the test of viral gene copy number in the liver of RVFV infected mice after treatment with the monoclonal antibody;
Figure 19 depicts the detection of viral gene copy number in the spleen of RVFV- infected mice after treatment with the monoclonal antibody;
Figure 20 shows the blocking effect of the monoclonal antibody on the binding of RVFV virions to Vero cells.

### SPECIFIC EMBODIMENTS

The present disclosure is further described with specific examples below, and the advantages and features of the present disclosure will become clearer with the description. However, these examples are only exemplary, and do not constitute any further limitations to the scope defined by the claims of the present disclosure.

### Example 1 Screening and preparation of the monoclonal antibody against RVF

### 1.1 Packaging of human recombinant adenovirus type 5 (HAdV5-GnGcopt) expressing GnGc protein of RVF virus

The recombinant protein GnGc coding gene (GenBank: DQ380208.1) is cloned into expression vector pDC316, which is then co-transfected into HEK293 cells with the backbone plasmid (pBHGlox_E1, Cre) of the AdMax adenovirus system. When the cytopathic effect is obvious, the cell supernatant is collected and inoculated into 293F cells for propagation, which is stored at -80 °C for further use after measurement of viral titer (see CN105483140A for construction method).

### 1.2 Immunization of rhesus monkey

Before immunization, 2 mL venous blood is collected from rhesus monkeys, and serum is separated as the negative serum control. Meanwhile, rhesus monkeys are immunized with 1 × 10⁸ IFU of HAdV5-GnGcopt intramuscularly. On Day 28 of immunization, rhesus monkeys are immunized again with HAdV5-GnGcopt at the same dose through the same method, and 2 mL blood is collected before immunization. On Days 56 and 182 of the first immunization, a mixture of 0.25 mg Gn protein and 0.25 mg Gc protein is thoroughly mixed with 0.5 mg aluminum adjuvant, to enhance the immunization of rhesus monkeys by intramuscular injection, and 2mL blood is collected before immunization.

### 1.3 Determination by ELISA on titers of antibodies in serum binding with Gn and Gc protein

Purified Gn and Gc proteins (GenBank: DQ380208.1) are coated in an ELISA plate (2 µg/mL, 100 µL/well) overnight at 4 °C, washed 3 times with PBST, sealed with 2% BSA at 37 °C for 1 hour; and washed 3 times. In the first well, the serum to be tested is diluted with PBST at a ratio of 1:100, then gradiently diluted at a ratio of 1:3, and incubated at 37 °C for 1 hour; washed 3 times, then added with HRP-labeled sheep anti-human secondary antibody, and incubated at 37 °C for 1 hour; washed 3 times, added with 100 µL TMB single component substrate solution, placed at room temperature for 5 minutes for color development, and added with 50 µL stop buffer; the value at OD450 nm-OD630 nm is determined by the ELISA reader. Finally, software GraphPad Prism is used to calculate the titer of Gn and Gc protein-binding antibodies.

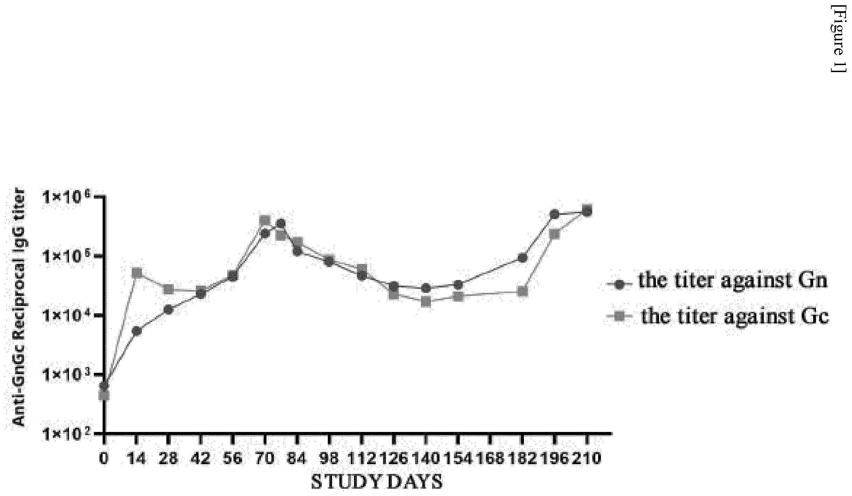

Within 28 days after the first immunization of rhesus monkeys with HAdV5-GnGcopt, the level of IgG binding antibodies against Gn and Gc proteins in rhesus monkeys show significant increase. Within 28 days after the second immunization with HAdV5-GnGcopt (Day 56 of the first immunization), the level of IgG binding antibodies against Gn and Gc proteins in rhesus monkeys increases further. On Day 56 of the first immunization, a mixture of Gn and Gc proteins is given to enhance the immunization of rhesus monkeys. Two weeks later, the levels of IgG binding antibodies against Gn and Gc proteins in rhesus monkeys reached a peak, slowly decreases within the following four weeks, and then remains stable. On Day 182 of the first immunization, a mixture of Gn and Gc proteins is provided to further strengthen the immunization of rhesus monkeys. Within the following four weeks, the level of IgG binding antibodies against Gn and Gc proteins in rhesus monkeys reaches a peak again, with further increased value compared with that on Day 70 of the first immunization. Throughout the entire immunization process, the level of the antibody against Gn protein in rhesus monkeys is generally slightly lower than that against Gc protein (see Figure 1).

### 1.4 Isolation of peripheral blood mononuclear lymphocytes from rhesus monkeys

10 mL anticoagulant blood is collected from the forelimbs of rhesus monkeys, and the whole blood is diluted with 10 mL PBS. 10 mL separation solution is added to a 50 mL centrifuge tube, and the diluted whole blood is slowly spread over the separation solution, to ensure a clear boundary between the two liquid layers. The centrifuge tube is slowly placed in a horizontal centrifuge whose acceleration and deceleration rates are set to the lowest level, and then centrifuged at 800 g at room temperature for 30 minutes. After centrifugation, the plasma is in the top layer, the separation solution in the middle layer, and the red blood cells at the bottom of the tube. The monocyte layer is located between the plasma layer and the separation solution layer. The monocytes are carefully aspirated into a new 50 mL centrifuge tube, added with 10 mL PBS and gently mixed to wash them. The centrifuge tube is then placed in a horizontal centrifuge, centrifuged at 300 g for 10 minutes. The supernatant is discarded. Then the mixture is washed again. The monocytes centrifuged to the bottom of the tube are resuspended with cell cryopreservation solution (10% DMSO and 90% FBS) and stored at -80 °C for later use.

### 1.5 Staining of Gn and Gc-specific memory B cells

The frozen PBMCs mentioned above is placed in a water bath at 37 °C to quickly thawed, then centrifuged at 500 g at room temperature for 5 minutes. The supernatant is discarded, and the cells are resuspended with 5 mL FPBS and transferred to a flow cytometry tube, and centrifuged at 500 g for 5 minutes. The supernatant is discarded. Washing is repeated. The supernatant is discarded. The cells are resuspended with 500 µL FPBS (2% FBS dissolved in PBS), and are counted. The specific dyeing process is as follows:
To modify the compensation of each fluorescence channel in the sorting process, cells are first treated with single fluorescence staining. Each of five flow cytometry tubes is added with 1 × 10⁵ cells, then, added with corresponding volumes of dye according to the required amount of dye provided in Table 1, including two tubes added with 2 µg each of Strep-tagged Gn and Gc proteins, supplemented with FPBS to 100 µL, gently mixed, and incubated at 4 °C in the dark for 30 minutes. Each tube is added with 3 mL FPBS, to gently resuspend and wash cells, then centrifuged at 500 g for 5 minutes. The supernatant is discarded, and cells are washed twice. Cells are resuspended with 100 µL FPBS, added with 1 µL APC-anti Strep-tag fluorescent antibody, gently mixed, and incubated at 4 °C in the dark for 30 minutes. After washing three times, the cells are resuspended with 1 mL FPBS for later use.

**Table 1. Volume of dye required for PBMCs staining**

| Name of dye | Volume of dye, /5×10⁵ cells |
|---|---|
| IgG (PE) | 10 µL |
| CD3 (PerCP) | 5 µL |
| CD19 (Alexfluor700) | 5 µL |
| Gn-Strep II tag (0.5 mg/mL) | 4 µL |
| Gc-Strep II tag (0.5 mg/mL) | 4 µL |

To perform single-cell flow cytometry sorting, cells are first treated with multi-fluorescence staining. 1 × 10⁵ cells are added into to a flow cytometry tube, supplemented with 5 dyes, including Gn and Gc proteins, according to the required dye amount provided in Table 1, gently mixed, and incubated at 4 °C in the dark for 30 minutes. 3 mL FPBS is added to gently resuspend and wash the cells, centrifuged at 500 g for 5 minutes. The supernatant is discarded, and cells are washed twice. Cells are resuspended with 100 µL FPBS, added with 2 µL APC-anti Strep-tag fluorescent antibody, gently mixed, and incubated at 4 °C in the dark for 30 minutes. After washing three times, the cells are resuspended with 1 mL FPBS for later use.

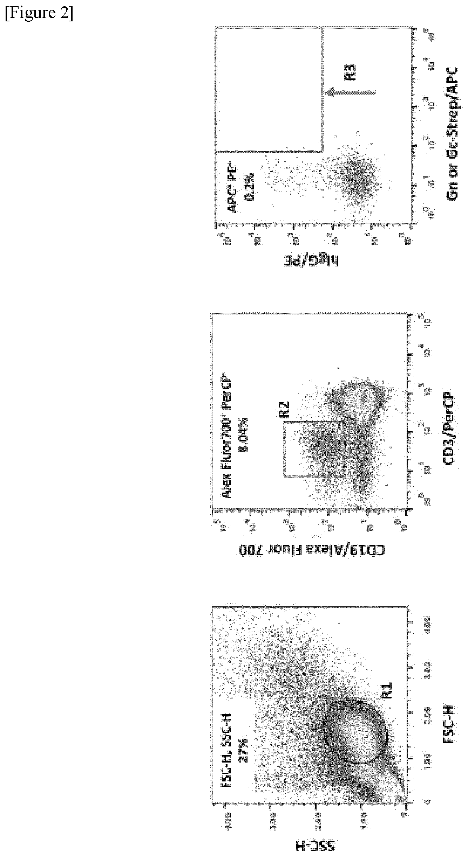

Continuous monitoring of the levels of Gn and Gc protein-binding antibodies in rhesus monkeys shows that at Week 4 of the fourth immunization, the levels of antibodies against Gn and Gc proteins in rhesus monkeys reach the peak. Therefore, blood samples at Week 4 of the fourth immunization are selected for single-cell sorting. Before sorting individual memory B cells by flow cytometry, PBMCs are isolated from peripheral blood of rhesus monkeys through density gradient centrifugation. Then, the single-cell flow cytometry Beckman MoFlo-XDP is used to sort individual Gn and Gc-specific memory B cells. In the process of sorting, lymphocytes, monocytes, and cell fragments are separated by forward scatter (FSC) and side scatter (SSC) (Figure 2, R1), with lymphocytes accounting for 27% of the total cells. Due to the expression of CD3 molecules on the surface of T cells rather than B cells, on the basis of R1, CD3 molecules are used to differentiate B cells from T cells. Also, since memory B cells express CD19 molecules, memory B cells are further circled by CD19 molecules (Figure 2, R2), which account for 8.04% of the total cells. Due to the expression of non-specific surface IgG and antigen-specific surface IgG on the surface of memory B cells, Gn and Gc-specific memory B cells are ultimately circled (Figure 2, R3), which accounted for approximately 0.2% of the total cells before sorting. Finally, 1,253 memory B cells are obtained through sorting.

### 1.6 Flow cytometry sorting of Gn and Gc-specific memory B cells

Before cells are sorted into a 96-well PCR plate, a mixture of 20 µL RNase-free distilled water and 20 Units RNase inhibitor is added to each well, and placed at 4 °C for later use.

Individual Gn and Gc-specific memory B cells are sorted. Cell samples are loaded after they are filtered through a 300-mesh cell sieve to remove masses formed by cell aggregation. The abovementioned cells treated with 5 single fluorescence staining are used for compensatory regulation of fluorescent dyes, followed by loading of cells stained with various dyes. Firstly, lymphocytes are selected and circled by FSC-H and SSC-H, and set as Gate 1. Based on the cells circled by Gate 1, CD19⁺/CD3⁻ cells are selected and circled through Alex Flour700 and PerCP, and set as Gate 2. Based on the cells circled by Gate 2, Gn⁺/Gc⁺/IgG⁺ cells are selected and circled by APC and PE, and set as Gate 3. The cell populations in Gate 3 (CD19⁺/CD3⁻/IgG⁺/Gn⁺/Gc⁺) are Gn and Gc-specific memory B cells. The sorting mode of the flow cytometer is set to "single-cell mode". Cells in Gate 3 are sorted into the 96-well PCR plate mentioned above, with one cell per well. After sorting, the 96-well PCR plate is sealed with a sealing plate film, immediately frozen in liquid nitrogen, and stored at -80 °C for future use.

### 1.7 Single cell PCR amplification of antibody variable region genes

### Single-cell reverse transcription PCR:

The reverse transcription kit SUPERSCRIPT III is used for reverse transcription PCR of individual memory B cells sorted above. The reaction system is shown in Table 2:

**Table 2. Single-cell reverse transcription PCR reaction system**

| **Component** | **Dose** |
|---|---|
| Template | 20 µL |
| Random primer | 3 µL |
| dNTP | 1 µL |
| 10×Buffer | 3 µL |
| 0.1M DTT | 1 µL |
| MgCl₂ | 2 µL |
| RNAase OUT | 1 µL |
| Super Script III | 0.5 µL |

The reaction procedure is shown in Table 3:

**Table 3. Single-cell reverse transcription PCR reaction procedure**

| Temperature | Time |
|---|---|
| 42 °C | 10 min |
| 25 °C | 10 min |
| 50 °C | 60 min |
| 94 °C | 5 min |
| 4 °C | End |

### Single-cell PCR amplification and screening of antibody genes encoding paired light and heavy chain:

### (1) First-round PCR amplification

Multiple H-chain primers in Table 6 are dissolved in ddH₂O, and mixed evenly with the same molar amount to form primer Mix. The κ chain primer and λ chain primer are mixed in the same way to form primer Mix. Then, the first-round PCR amplification of antibody genes starts by using TranStart TaqDNA polymerase, with the reverse transcription PCR product as the template, and primer Mix in Table 6 as the primer.

The reaction system is shown in Table 4:

**Table 4. First-round PCR amplification reaction system**

| Component | Dose |
|---|---|
| Template | 2 µL |
| Primer Mix (H/κ/λ) | Each primer of 20 pmol |
| dNTP | 4 µL |
| 10×Buffer | 5 µL |
| TranStart Taq DNA polymerase | 0.5 µL |
| ddH₂O | up to 50 µL |

The reaction procedure is shown in Table 5:

**Table 5. First-round PCR amplification reaction procedure**

| Temperature | Time |
|---|---|
| 95 °C | 5 min |
| 95 °C | 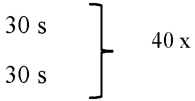 |
| 55 °C | |
| 72 °C | 45 s |
| 72 °C | 10 min |
| 16 °C | End |

### (2) Second-round PCR amplification

Multiple H-chain primers in Table 7 are dissolved in ddH₂O, and mixed evenly with the same molar amount to form primer Mix. The κ chain primer and λ chain primer are mixed in the same way to form primer Mix. Then, the second-round PCR amplification of antibody genes starts by using TranStart TaqDNA polymerase, with the first-round PCR product as the template, and primer Mix in Table 7 as the primer. The reaction system and procedure are the same as the first-round PCR.

**Table 6. First-round PCR primers**

| Name of primer | Sequence (5'-3') |
|---|---|
| H-chain primers | |
| 5'VH1.L1 | ATGGACTKGACCTGGAGG |
| 5'VH2.L1 | ATGGACACGCTTTGCTCC |
| 5'VH3A.L1 | ATGGAGTTKGGGCTGAGCTG |
| 5'VH3B.L1 | ATGGAGTTTGKRCTGAGCTGG |
| 5'VH3C.L1 | ATGGAGTCRTGGCTGAGCTGG |
| 5'VH3D.L1 | ATGGAGTTTGTGCTGAGTTTGG |
| 5'VH4.L1 | ATGAAGCACCTGTGGTTC |
| 5'VH5A.L1 | ATGGGGTCAACTGCCATC |
| 5'VH5B.L1 | ATGGGGTCCACCGTCACC |
| 5'VH6.L1 | ATGTCTGTCTCCTTCCTCA |
| 5'VH7.L1 | ATGGACCTCACCTGGAGC |
| 3'IgG (Outer) | GGAAGGTGTGCACGCCGCTGGTC |

| κ-chain primers | |
|---|---|
| 5'VK1A.L1 | ATGGACATGAGGGTCCCCGC |
| 5'VK1B.L1 | GGCTCCTKCTGCTCTGGCTC |
| 5'VK2.L1 | ATGARGYTCCCTGCTCAG |
| 5'VK3.L1 | ATGGAARCCCCAGCWCAGC |
| 5'VK4.L1 | ATGGTGTCACAGACCCAAGTC |
| 5'VK5.L1 | ATGGCATCCCAGGTTCASC |
| 5'VK6A.L1 | ATGTTGTCTCCATCACAACTC |
| 5'VK6B.L1 | ATGGTGTCCCCATTGCAACTC |
| 5'VK7.L1 | ATGGGGTCCTGGGCTCC |
| 3'Kappa (Outer) | GTCCTGCTCTGTGACACTCTC |

| λ-chain primers | |
|---|---|
| 5'VL1.L1 | ATGGCCTGGTYYCCTCTC |
| 5'VL2/7/10.L1 | ATGGCCTGGRCTCTGCTCC |
| 5'VL3A.L1 | ATGGCCTGGATTCCTCTC |
| 5'VL3B.L1 | ATGGCCTGGACCTTTCTC |
| 5'VL3C.L1 | ATGGCCTGGACCCCTCCC |
| 5'VL4A.L1 | ATGGCCTGGGTCTCCTTC |
| 5'VL4B.L1 | ATGGCCTGGACCCCACTC |
| 5'VL5/11.L1 | ATGGCCTGGACTCCTCTC |
| 5'VL6.L1 | ATGGCCTGGGCTCCACTCC |
| 5'VL8.L1 | ATGGCCTGGATGATGCTTC |
| 5'VL9.L1 | ATGGCCTGGGCTCCTCTG |
| 3'Lambda (Outer) | TGTTGCTCTGTTTGGAGGG |

**Table 7. Second-round PCR primers**

| Name of primer | Sequence (5'-3') |
|---|---|
| H-chain primers | |
| 5'VH1A.SE | TGGCAGCAGCTACAGGTGC |
| 5'VH1B.SE | TGACAGCAGCTACAGGCGC |
| 5'VH1C.SE | TGGCAGCAGCAACAGGCAC |
| 5'VH2.SE | GTCCCGTCCTGGGTCTTGTC |
| 5'VH3A.SE | GCTGTTTGGAGAGGTGTCCAGTGTG |
| 5'VH3B.SE | GCCATATTAGAAGGTGTCCAGTGTG |
| 5'VH3C.SE | GCTCTTTTGAAAGGTGTCCAGTGTG |
| 5'VH3D.SE | GCTATTTTAAGAGGTGTCCAGTGTG |
| 5'VH3E.SE | GCTATTTTAAAAGGTGTCCAGTGTG |
| 5'VH4.SE | AGCTCCCAGATGGGTCYTGTCC |
| 5'VH5.SE | GCTGTTCTCCARGGAGTCTGTG |
| 5'VH6.SE | GGCCTCCCATGGGGTGTC |
| 5'VH7A.SE | GCAGCAACAGGTGCCCACTC |
| 5'VH7B.SE | GCAGCAACAGGCACCCACTC |
| 3'IgG (Inner) | GTTCAGGGAAGTAGTCCTTGAC |

| κ-chain primers | |
|---|---|
| 5'VK1/2.SE | CTCCCAGGTGCCAGATGTGA |
| 5'VK1B.SE | GGTCCCTGGRTCCAGTGGG |
| 5'VK3A.SE | TGGCTCCCAGGTACCACYGGA |
| 5'VK3B.SE | TGGATCCCGGATGCCGCCG |
| 5'VK3C.SE | TGGCTTCCGGATACCACTGGA |
| 5'VK4.SE | CTGGATCTCTGGTGTCTGTGG |
| 5'VK5.SE | CCTTTGGATCTCTGMTGCCAGG |
| 5'VK6.SE | TGGGTTCCAGTCTCCAAGGG |
| 5'VK7.SE | TGTGCTCCAGGCTGCAATGG |
| 3'Kappa (Inner) | ATTCAGCAGGCACACAACAGAG |

| λ-chain primers | |
|---|---|
| 5'VL1A.SE | CTGTGCAGGGTCCTGGGCC |
| 5'VL1B.SE | CTGCACAGGGTCCYGGGCC |
| 5'VL2.SE | TCACTCAGGGCACAGGATCC |
| 5'VL3A.SE | CGCCCTCTGCACAGTCTCTGTGG |
| 5'VL3B.SE | CACTCTCTGCACAGGTTCCGTGG |
| 5'VL4A.SE | TTCATTTTCTCCACAGGTCTCTGTG |
| 5'VL4B.SE | CTTCACTGCAGAGGTGTCTCTC |
| 5'VL5.SE | CACTGCACAGGTTCCCTCTC |
| 5'VL6.SE | CTGCACAGGGTCTTGGGCTG |
| 5'VL8.SE | GCTTATGGCTCAGGAGTGGA |
| 3'Lambda(Inner) | AGACACACTAGTGTGGCCTTG |

### (3) Screening of antibody genes encoding paired light and heavy chain

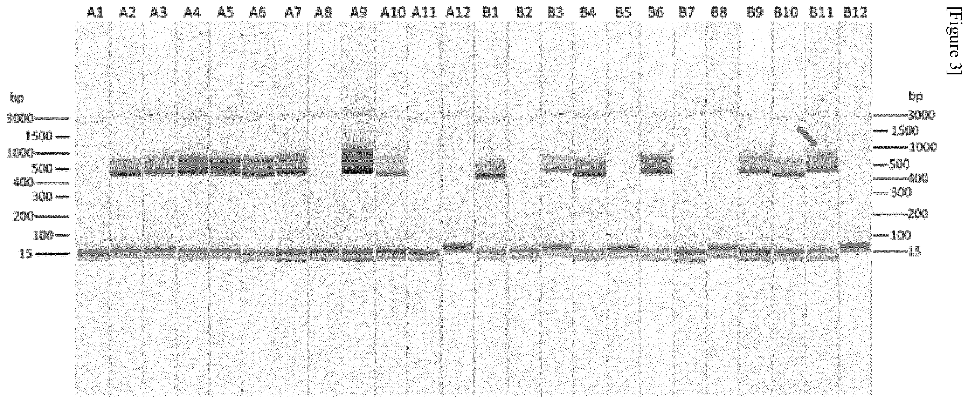

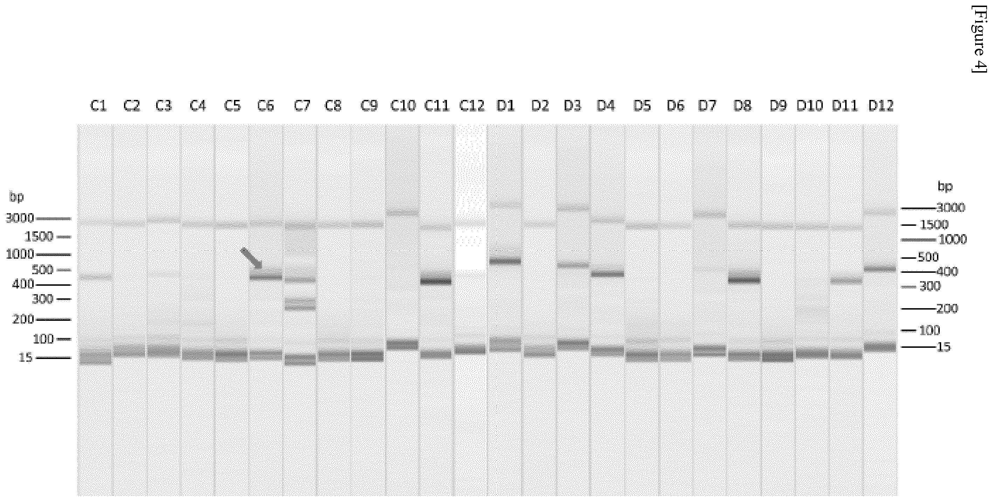

Capillary electrophoresis of nucleic acid is performed for products amplified by two rounds of PCR using QIAxcel DNA Fast Analysis Kit. Single cell clones with positive light and heavy chains are recorded as successfully paired positive clones, and the corresponding PCR products are selected and stored at -20 °C. The two-round nested PCR amplification band of antibody A38 heavy chain is consistent with the expected size, as shown in Lane B11, Figure 3; The two-round nested PCR amplification band of the light chain is also in line with the expected size, as revealed in Lane C6, Figure 4.

### PCR amplification of antibody genes encoding paired light and heavy chain variable region:

Firstly, multiple H-chain variable region amplification primers in Table 9 are dissolved in ddH₂O, and mixed evenly with the same molar amount to form primer Mix. The κ chain primer and λ chain primer are mixed in the same way to form primer Mix. Then, PCR amplification of the antibody variable region genes starts by using TranStart TaqDNA polymerase, with PCR products of the successfully paired light and heavy chains mentioned above as the template, and primer Mix in Table 9 as the primer. The reaction system is the same as nested PCR. The reaction procedure is shown as Table 8:

**Table 8. Procedure of PCR amplification reaction for variable region genes**

| Temperature | Time |
|---|---|
| 95 °C | 5 min |
| 95 °C | 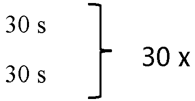 |
| 55 °C | |
| 72 °C | 45 s |
| 72 °C | 10 min |
| 16 °C | End |

**Table 9. Variable region amplification primers**

| Name of primer | Sequence (5'-3') |
|---|---|
| H-chain variable region amplification primers | |
| 5'VH1/5/7 | CTAATTTTAAAAGGTGTCCAGTGTGAGGTGCAG CTGGTGCAG |
| 5'VH3 | CTAATTTTAAAAGGTGTCCAGTGTGAGGTGCAG CTGGTGGAG |
| 5'VH3-23 | CTAATTTTAAAAGGTGTCCAGTGTGAGGTGCAG CTGTTGGAG |
| 5'VH4 | CTAATTTTAAAAGGTGTCCAGTGTCAGGTGCAG CTGCAGGAG |
| 5'VH4-34 | CTAATTTTAAAAGGTGTCCAGTGTCAGGTGCAG CTACAGCAGTG |
| 5'VH1-18 | CTAATTTTAAAAGGTGTCCAGTGTCAGGTTCAG CTGGTGCAG |
| 5'VH1-24 | CTAATTTTAAAAGGTGTCCAGTGTCAGGTCCAG CTGGTACAG |
| 5'VH3-9/30/33 | CTAATTTTAAAAGGTGTCCAGTGTGAAGTGCAG CTGGTGGAG |
| 5'VH6-1 | CTAATTTTAAAAGGTGTCCAGTGTCAGGTACAG CTGCAGCAG |
| 3'JH1/2/4/5 | GGGGAAGACCGATGGGCCCTTGGTCGACGCTGA GGAGACGGTGACCAG |
| 3'JH3 | GGGGAAGACCGATGGGCCCTTGGTCGACGCTGA AGAGACGGTGACCATTG |
| 3'JH6 | GGGGAAGACCGATGGGCCCTTGGTCGACGCTGA GGAGACGGTGACCGTG |

| κ-chain variable region amplification primers | |
|---|---|
| 5'Vκ1 | CTATGGGTATCTGGTACCTGTGGGGACATCCAG ATGACCCAGTC |
| 5'Vκ1-9/1-13 | CTATGGGTATCTGGTACCTGTGGGGACATCCAG TTGACCCAGTCT |
| 5'Vκ1D-43/1-8 | CTATGGGTATCTGGTACCTGTGGGGCCATCCGG ATGACCCAGTC |
| 5'Vκ2 | CTATGGGTATCTGGTACCTGTGGGGATATTGTG ATGACCCAGAC |
| 5'Vκ2-28/2-30 | CTATGGGTATCTGGTACCTGTGGGGATATTGTG ATGACTCAGTC |
| 5'Vκ3-11/3D-11 | CTATGGGTATCTGGTACCTGTGGGGAAATTGTG TTGACACAGTC |
| 5'Vx3-15/3D-15 | CTATGGGTATCTGGTACCTGTGGGGAAATAGTG ATGACGCAGTC |
| 5'Vκ3-20/3D-20 | CTATGGGTATCTGGTACCTGTGGGGAAATTGTG TTGACGCAGTCT |
| 5'Vκ4-1 | CTATGGGTATCTGGTACCTGTGGGGACATCGTG ATGACCCAGTC |
| 3'Jκ1/2/4 | GAAGACAGATGGTGCAGCCACAGTACGTTTGAT YTCCACCTTGGTC |
| 3'Jκ3 | GAAGACAGATGGTGCAGCCACAGTACGTTTGAT ATCCACTTTGGTC |
| 3'Jκ5 | GAAGACAGATGGTGCAGCCACAGTACGTTTAAT CTCCAGTCGTGTC |

| λ-chain variable region amplification primers | |
|---|---|
| 5'Vλ1 | CTATGGGTATCTGGTACCTGTGGGCAGTCTGTG CTGACKCAG |
| 5'Vλ2 | CTATGGGTATCTGGTACCTGTGGGCAGTCTGCC CTGACTCAG |
| 5'Vλ3 | CTATGGGTATCTGGTACCTGTGGGTCCTATGAG CTGACWCAG |
| 5'Vλ4/5 | CTATGGGTATCTGGTACCTGTGGGCAGCYTGTG CTGACTCA |
| 5'Vλ6 | CTATGGGTATCTGGTACCTGTGGGAATTTTATG CTGACTCAG |
| 5'Vλ7/8 | CTATGGGTATCTGGTACCTGTGGGCAGRCTGTG GTGACYCAG |
| 3'JL-1 | GGGGGCAGCCTTGGGCTGACG TAGGACGGTCA GCCGGGTCCC |
| 3'JL-2 | GGGGGCAGCCTTGGGCTGACG TAGGACGGTCA GCCGAGTCCC |

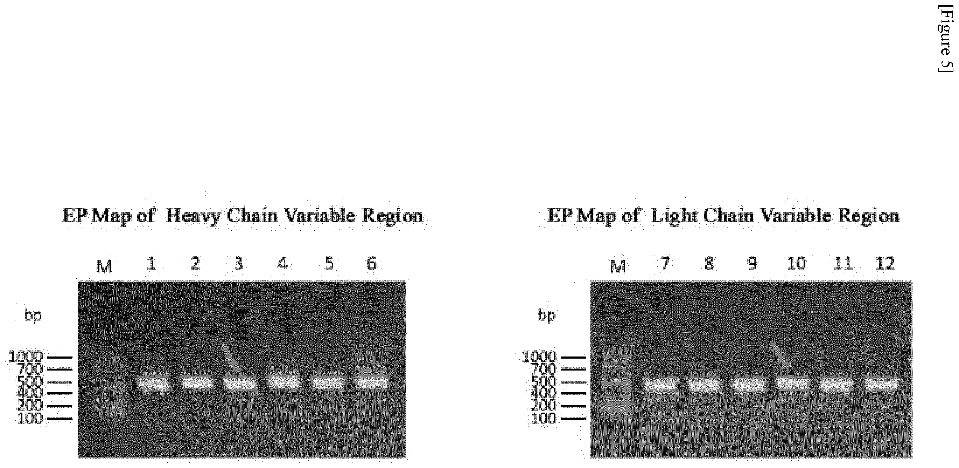

To amplify antibody genes from Gn-specific memory B cells sorted above, firstly, reverse transcription kit are used to obtain cDNA encoding antibody from 564 memory B cells. Due to the quantity of transcripts of antibody genes in individual memory B cells is too low, the antibody genes in memory B cells are amplified through two rounds of PCR with specific primers of rhesus monkey antibody genes, to screen paired light and heavy chain antibodies. A total of 273 antibodies with successfully paired light and heavy chains are identified, including 162 antibodies with κ light chain, and 111 antibodies with λ light chain, with the total pairing success rate of about 48.4%. To clone the variable regions of light and heavy chains of the screened rhesus monkey monoclonal antibody with the constant regions of the humanized IgG light and heavy chains, the products of nested second-round PCR from the single-cell successfully paired light and heavy chains are selected, and used as the template to amplify the antibody variable region genes using specific antibody primers. Results show that the antibody heavy and light chain variable regions is consistent with expected size, with amplification efficiency of 100%. The PCR amplification band of A38 heavy chain variable region (about 450 bp) is in line with the expected results, as shown in Lane 3, Figure 5, and the PCR amplification band of light chain variable region (about 450 bp) is consistent with the expected size, as shown in Lane 10, Figure 5.

### 1.8 Construction of a linear expression cassette for monoclonal antibody genes

The antibody variable region genes are cloned between the leader sequence, containing the promoter and signal peptide, and the constant region-poly (A) tail fragment, containing the antibody constant region and terminator, by overlap extension PCR.

### Amplification of the leader sequence:

The leader sequence of antibody heavy chain genes is amplified by upstream and downstream primer CMV-UP/3'Leader H, with plasmid pCDNA-H as the template; The leader sequence of antibody light chain genes is amplified by upstream and downstream primer CMV-UP/3'Leader L, with pCDNA-λ as the template. The primer sequence is listed in Table 10. The reaction system and reaction procedure are the same as variable region amplification PCR.

**Table 10. Leader sequence amplification primers**

| Name of primer | Sequence (5'-3') |
|---|---|
| CMV-UP | GATATACGCGTTGACATTGATTATTGAC |
| 3'Leader H | ACACTGGACACCTTTTAAAATTAG |
| 3'Leader L | CCCACAGGTACCAGATACCCATAG |

### Amplification of constant region-poly (A) tail fragment:

The constant region-poly (A) tail fragment of the antibody heavy chain gene is amplified by upstream and downstream primer 5'CH/TK-POLYA, with plasmid pCDNA-H as the template; The constant region-poly (A) tail fragment of the antibody κ chain gene is amplified by upstream and downstream primer 5'Cκ/TK-POLYA, with pCDNA-κ as the template; The constant region-poly (A) tail fragment of the antibody λ chain gene is amplified by upstream and downstream primer 5'Cλ/TK-POLYA, with pCDNA-λ as the template. The primer sequences are shown in Table 11. The reaction system and reaction procedure are the same as variable region amplification PCR.

**Table 11. Constant region-poly (A) tail fragment amplification primers**

| Name of primer | Sequence (5'-3') |
|---|---|
| 5'CH | ACCAAGGGCCCATCGGTCTTCCCC |
| 5'Cλ | ACTGTGGCTGCACCATCTGTCTTC |
| 5'Cλ | CGTCAGCCCAAGGCTGCCCCC |
| TK-POLYA | AAGTGTAGCGGTCACGCTGCGCGTAACC |

### Amplification of linear expression cassette of antibody genes:

The linear expression cassettes of antibody H, κ and λ chain full-length genes are amplified by overlap extension PCR, respectively, with abovementioned amplified leader sequences, variable region genes of antibody light and heavy chains, and constant region-poly (A) tail fragments as templates, and CMV-UP/TK-POLYA as the upstream and downstream primer. The reaction procedure is the same as variable region amplification PCR. The reaction system is shown in Table 12:

**Table 12. Amplification reaction system of linear expression cassette of antibody genes**

| Component | Dose |
|---|---|
| Leader sequence | 10 ng |
| Antibody variable region genes | 1 µL |
| Constant region-poly (A) tail fragment | 10 ng |
| CMV-UP | 1 µL |
| TK-POLYA | 1 µL |
| dNTP | 4 µL |
| 10×Buffer | 5 µL |
| TranStart Taq polymerase | 0.5 µL |
| ddH₂O | up to 50 µL |

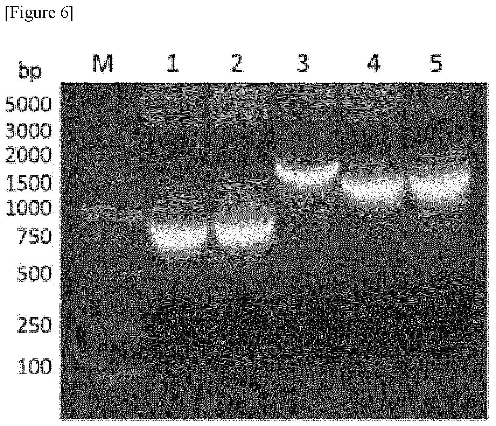

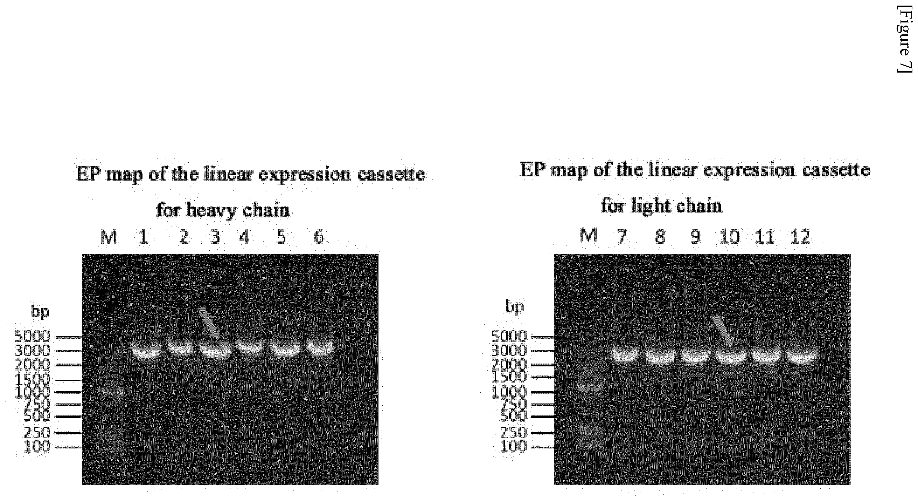

In order to clone the amplified antibody light and heavy chain variable region with the humanized IgG light and heavy chain constant region, and to quickly screen the antibodies binding with Gn and Gc proteins, the antibody light and heavy chain variable region genes are cloned by overlap extension PCR between the leader sequence, containing the CMV promoter, and signal peptide, and the constant region-poly (A) tail fragment, containing the antibody constant region and terminator, to form the linear expression cassette containing the antibody light and heavy chain full-length genes. Firstly, the corresponding leader sequences and constant region-poly (A) tail fragments of antibody H, κ and λ chains are successfully amplified, with the size of the leader sequences of both heavy and light chains of 750 bp (Lanes 1 and 2, Figure 6), the heavy chain constant region-poly (A) tail fragments of 1,900 bp (Lane 3, Figure 6), and κ & λ chain constant region-poly (A) tail fragments of 1,350 bp (Lanes 4 and 5, Figure 6). Then, the linear expression cassette of A38 antibody light and heavy chains is successfully amplified through overlap extension PCR, with the size of the linear expression cassette of the heavy chain of about 3,200 bp (Lane 3, Figure 7), and the linear expression cassette of the light chain of approximate 2,500 bp (Lane 10, Figure 7).

### 1.9 Recovery and purification of linear expression cassette of monoclonal antibody genes

To achieve high-throughput purification of the PCR product of the linear expression cassette of the antibody genes amplified above, N96 DNA product purification kit from Tiangen Biotech Co., Ltd. is used for gel recovery. The specific steps are as follows:
1) Equilibrium of 96-well adsorption plate: the 96-well adsorption plate CB2 is placed on the 96-well deep well plate, 500 µL buffer BL is added to each well, and placed in a horizontal centrifuge, centrifuged at 3,500 rpm at room temperature for 4 minutes. The waste liquid in the deep well plate is discarded, and the adsorption plate is repositioned on the deep well plate.
2) The PCR product of linear expression cassette is evenly mixed with the buffer PB at a ratio of 1:3, and placed at room temperature for 3 minutes.
3) The mixture is transferred to the balanced adsorption plate, centrifuged at 3,500 rpm at room temperature for 6 minutes. The waste liquid in the deep well plate is discarded, and the adsorption plate is repositioned on the deep well plate.
4) 700 µL buffer PW is added to the centrifuged adsorption plate, centrifuged at 3,500 rpm at room temperature for 5 minutes. The waste liquid in the deep well plate is discarded, the adsorption plate is repositioned on the deep well plate. Washing is repeated once.
5) The washed adsorption plate is centrifuged at 3,500 rpm at room temperature for 10 minutes, and placed at room temperature for 5 minutes to remove any remaining washing buffer.
6) The adsorption plate is placed in a new deep well plate, and 80 µL ddH₂O is added to the center of the spin column, placed at room temperature for 5 minutes, and centrifuged at 3,500 rpm for 15 minutes. The purified PCR products of linear expression cassette are collected, with concentration measured by the UV spectrophotometer, and stored at -20 °C for future use.

### 1.10 Co-transfection of paired antibody gene linear expression cassette into 293T cells

24 hours before transfection, 293T cells are spread in the 96-well cell plate at a density of 4 × 10⁴ cells/well, and incubated overnight in a cell incubator at 37 °C under 5% CO₂ atmosphere. Then, the paired light and heavy chain linear expression cassettes are added as 0.1 µg/well to 20 µL Opti-MEM medium, and gently mixed. TurboFect transfection reagent is added as 0.4 µL/well to the diluted linear expression cassettes, and incubated at room temperature for 18 minutes. The incubated mixture is gently added into the 96-well cell plate with a pipettor, and incubated in a cell incubator at 37 °C under 5% CO₂ for 48 hours.

### 1.11 ELISA rapid screening of binding antibodies against Gn protein

The purified truncated Gn protein is coated in an ELISA plate (2 µg/mL, 100 µL/well) overnight at 4 °C, washed three times with PBST, and sealed with 2% BSA at 37 °C for 1 hour; washed three times with PBST. 50 µL 293T cell expression supernatant is added to each well, supplemented with diluent to 100 µL, incubated at 37 °C for 1 hour, and washed three times with PBST. 100 µL HRP-labeled sheep anti-human IgG secondary antibody (diluted at 1:10,000) is added to each well, incubated at 37 °C for 1 hour, and washed three times with PBST. 100 µL TMB single component substrate solution is added to each well, placed at room temperature for 5 minutes for color development. 50 µL stop buffer is added, and the value at OD450 nm-OD630 nm is finally determined by the ELISA reader.

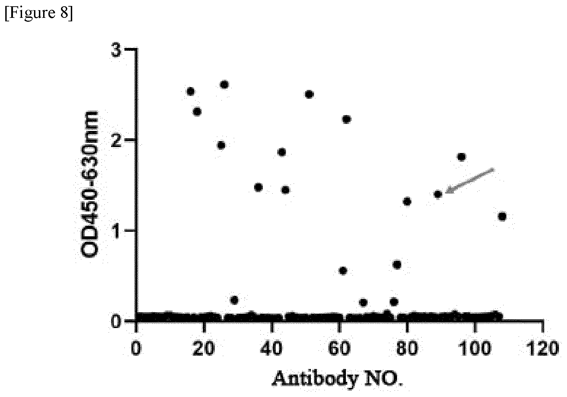

To quickly screen the binding antibodies against Gn protein, 273 successfully amplified light and heavy chain linear expression cassettes are co-transfected into 293T cells, and incubated for 48 hours. The supernatant is collected. ELISA is used to detect the presence of binding antibodies against Gn protein in the supernatant, 33 binding antibodies against Gn protein are identifies, including 18 antibodies with κ light chains, and 15 antibodies with λ light chains, with the total positive rate of antibodies against Gn protein of 12.08%. The OD value of A38 binding is 1.4023, as shown in Figure 8.

### 1.12 Construction of eukaryotic expression plasmid for Gn protein -binding antibody

To improve the expression of Gn protein - binding antibody, the positive antibody light and heavy chain full-length genes are cloned into the eukaryotic expression plasmid pCDNA3.4 through homologous recombination.

### Amplification of the positive antibody light and heavy chain full-length genes:

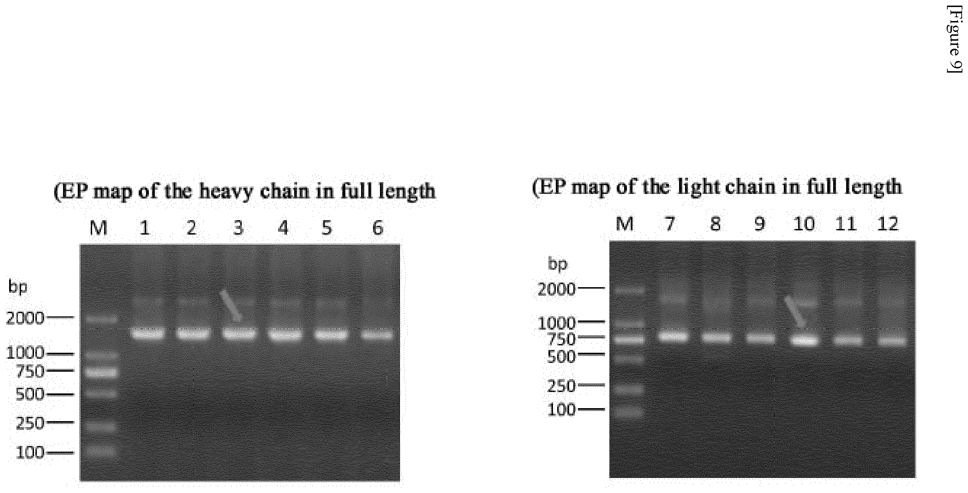

The antibody light and heavy chain full-length genes are amplified by upstream and downstream primer AbHR-F/AbHR-R, with the linear cassette of the above-mentioned positive antibody light and heavy chains as the template. The primer sequences are shown in Table 13. The reaction system and procedure are the same as variable region amplification PCR, and the size of A38 antibody heavy and light chain full-length PCR is consistent with the expectation. Results are shown in Lanes 3 and 10, Figure 9. The amplified PCR products are recovered and purified.

**Table13 Amplification primers of positive antibody light and heavy chain full-length genes**

| Name of primer | Sequence (5'-3') |
|---|---|
| AbHR-F | CCTTGGATCTCTAGCGAATTCAATTGCCGCCACCATG |
| AbHR-R | CTTGTCGAGGTCGGGGGATCCGGCCTTGCCGGCCTCGAG |

### Linearization by double enzyme digestion and recovery & purification of eukaryotic expression plasmid pCDNA3.4

The plasmid pCDNA3.4 is linearized by double enzyme digestion with restriction endonucleases EcoR I and BamH I. The enzyme digestion system is shown in Table 14:

**Table 14. Plasmid pCDNA3.4 enzyme digestion reaction system**

| Component | Dose |
|---|---|
| pCDNA3.4 | 3 µg |
| EcoR I | 3 µL |
| BamH I | 3 µL |
| Buffer | 10 µL |
| ddH₂O | up to 100 µL |

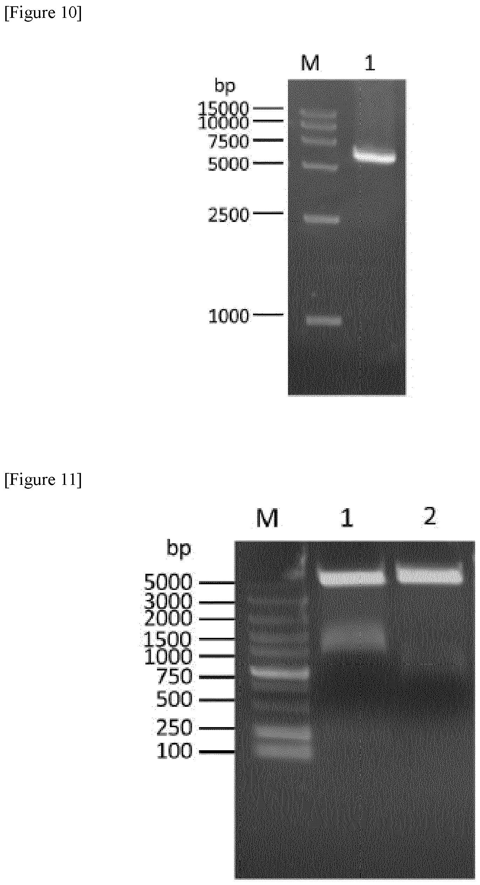

The plasmid is digested by enzyme at 37 °C for 2 hours, with results shown in Figure 10, followed by gel recovery and purification, and stored at -20 °C for later use.

### Litigation, transformation, and screening for positive clone:

According to the instructions, the NEBuilder assemble kit is used to litigate the target fragment and the vector. The reaction system is shown in Table 15

**Table 15. NEBuilder assemble system**

| Component | Dose |
|---|---|
| Heavy chain genes/light chain genes | 48 ng/24 ng |
| pCDNA3.4 | 100 ng |
| NEBuilder | 5 µL |
| ddH₂O | up to 10 µL |

The system is mixed slightly, litigated at 50 °C for 15 minutes, and placed on ice. 2 µL litigated product is added to the Top10 competent cells, placed into an ice bath for 30 minutes, heat shocked at 42 °C for 90 seconds, placed into an ice bath for 3 minutes. Non-resistant LB medium is used, shaking cultured for 60 minutes, and centrifuged at 8,000 rpm for 1 minute. The precipitate is resuspended, and 100 µL resuspension is coated on the plate, and incubated at 37 °C overnight in an incubator. A sterile pipettor tip is used to select monoclonal colonies with good growth state and transfer them to a 1.5 mL aseptic centrifuge tube containing 600 µL Amp/LB liquid medium, incubated in a shaker at 37 °C for 12 hours. The cultured bacterial solution is identified by PCR with corresponding primers, and the positive monoclonal colonies are verified by sequencing.

### Identification of A38 eukaryotic expression plasmid by double enzyme digestion

Restriction endonucleases EcoR I and BamH I are used for identification of double enzyme digestion of A38 light and heavy chain expression plasmid. The enzyme digestion system is shown in Table 16:

**Table 16. Double enzyme digestion system of A38 light and heavy chain expression plasmid**

| Component | Dose |
|---|---|
| pCDNA3.4 | 3 µg |
| EcoR I | 3 µL |
| BamH I | 3 µL |
| Buffer | 10 µL |
| ddH₂O | up to 100 µL |

Electrophoresis is performed after 2 hours of enzyme digestion at 37 °C, and the identification results are shown in Figure 11, with Lane 1 indicating the double enzyme digestion results of A38 antibody heavy chain plasmid, and Lane 2 showing the double enzyme digestion results of A38 antibody light chain plasmid.

### 1.13 Eukaryotic expression and purification of antibodies binding with Gn protein

The paired light and heavy chain plasmids whose sequence is confirmed are co-transfected into Expi293F cells according to the instructions. The supernatant is collected after incubation for 108 hours, centrifuged at 1,500 g at 4 °C for 15 minutes, and then centrifuged at 3,000 g for 15 minutes. The supernatant is transferred to a new centrifuge tube, and centrifuged at high speed (12,000 rpm) for 10 minutes. The supernatant after high-speed centrifugation is transferred to a new centrifuge tube, and placed at 4 °C for later use.

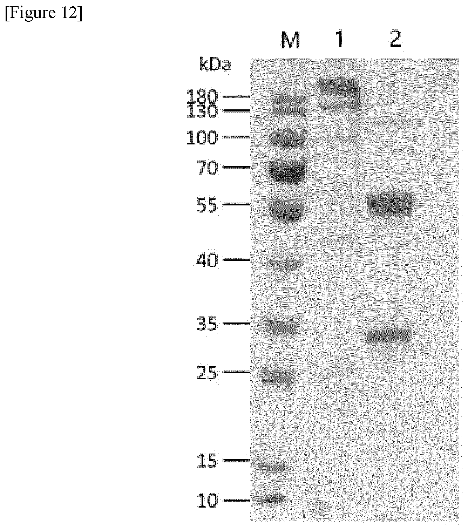

To isolate the antibodies expressed by Expi293F cells from the cell culture medium, the antibodies are purified by HiTrap rProteinA affinity column, with steps shown as follows:
1) Firstly, the HiTrap rProteinA affinity column is connected to the protein purifier AKTA pure at a low flow rate, and 20% ethanol in the rProteinA affinity column is washed with 5 column volumes of purified water. The equilibrium buffer (PBS) is used to balance the column, until UV is plat. Then, the UV is set to zero.
2) After high-speed centrifugation, the cell supernatant is loaded at a normal flow rate, and the column is collected. After loading, PBS is used to balance the column until UV is flat.
3) The antibody in the affinity column is eluted with 0.1 M glycine at pH 2.7, and the elution peak is collected. The eluent is neutralized with Tris-HCl buffer from pH 9.0 to pH 7.0. SDS-PAGE is used to analyze the purified antibody in the reduced and non-reduced condition. A38 antibody has high expression and good purity, as shown in Figure 12, with Lane 1 indicating the purified A38 antibody under the non-reduced condition, and Lane 2 showing the purified A38 antibody under the reduced condition.
4) The purified antibody is concentrated and buffer exchanged into PBS by a 50kDa ultrafiltration tube, then centrifuged at 3,000 g at 4 °C for 30 minutes. Finally, the concentration is determined by the BCA assay kit, and about 1.2 mg antibody is obtained from 30 mL suspension cells.

### 1.14 Binding activity of purified monoclonal antibody against Gn protein determined by ELISA

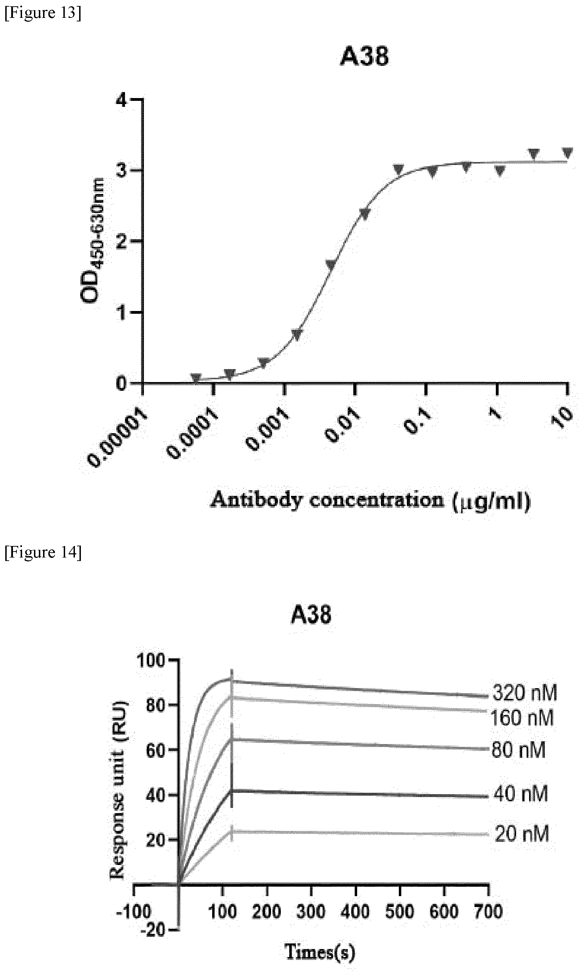

The purified truncated Gn protein is coated in an ELISA plate (2 µg/mL, 100 µL/well) overnight at 4 °C, washed three times with PBST, and sealed with 2% BSA at 37 °C for 1 hour; washed three times with PBST. 150 µL antibody at a concentration of 10 µg/mL is added to the first well, followed by twelve 3-fold gradient dilutions, incubated at 37 °C for 1 hour, and washed three times with PBST. 100 µL HRP-labeled sheep anti-human IgG secondary antibody (diluted at 1:10,000) is added to each well, incubated at 37 °C for 1 hour, and washed three times with PBST. 100 µL TMB single component substrate solution is added to each well, placed at room temperature for 5 minutes for color development. 50 µL stop buffer is added, and the value at OD450 nm-OD630 nm is determined by the ELISA reader. Software Graphpad Prism is used for four-parameter fitting of the binding curve of each monoclonal antibody. A38 shows good binding activity with Gn protein, with EC₅₀ of 4.515 ng/mL, with results shown in Figure 13.

### 1.15 the sequencing result of monoclonal antibody A38 is shown as follow:

The amino acid sequence of the heavy chain variable region is shown as SEQ ID NO.1, wherein the amino acid sequence of CDR1, CDR2 and CDR3 in the heavy chain variable region is shown as the positions 27-35, 53-60, 99-112 of SEQ ID NO:1 respectively. The sequence of the polynucleotide encoding the heavy chain variable region is shown as SEQ ID NO:2. The amino acid sequence of the heavy chain constant region of the antibody is shown as SEQ ID NO:3, and the sequence of the polynucleotide encoding the heavy chain constant region of the antibody is shown as SEQ ID NO:4. The heavy chain is human-derived.

The amino acid sequence of the light chain variable region is shown as SEQ ID NO.5, wherein the amino acid sequence of CDR1, CDR2 and CDR3 in the heavy chain variable region is shown as the positions 26-34, 52-58, and 97-106 of SEQ ID NO:5 respectively. The sequence of the polynucleotide encoding the light chain variable region is shown as SEQ ID NO:6. The light chain constant region of the antibody of the present disclosure can optionally be κ chain constant region or λ chain constant region, wherein the amino acid sequence of the κ chain constant region is shown as SEQ ID NO:7, and the sequence of the polynucleotide encoding the κ chain constant region is shown as SEQ ID NO:8. The amino acid sequence of the λ chain constant region is shown as SEQ ID NO:9, and the sequence of the polynucleotide encoding the λ chain constant region is shown as SEQ ID NO:10. The both light chain is human-derived. In a specific embodiment of the present disclosure, the λ chain constant region is optionally as the light chain constant region of the monoclonal antibody A38 to undergo the following pharmacodynamics experiment.

### Example 2. Pharmacodynamic study

### 2.1 Determination of neutralizing antibody affinity

The affinity between A38 and Gn protein is determined by Protein A chip using Biacore T200 instrument. The antibody is diluted to 0.5 µg/mL, and captured by Protein A chip at a flow rate of 10 µL/min. The Gn protein is diluted to 320 nM, 160 nM, 80 nM, 40 nM, and 20 nM, and tested through Protein A chip that having captured A38 at a flow rate of 30 µL/min, to determine the binding rate (Kₐ) by binding for 120s and the dissociation rate (K_{d}) by dissociating for 600s. The affinity (K_{D}) of the tested antibody is calculated by the ratio of Kₐ to K_{d}. The affinity between A38 and Gn protein is measured to be 0.97 nM, as showed in Figure 14 and Table 17, which show fast binding rate and slow dissociation rate of A38 with Gn protein, indicating good affinity between A38 and Gn protein.

**Table 17. Analysis of affinity between A38 antibody and Gn protein**

| kₐ (1/Ms) | k_{d} (1/s) | K_{D} (nM) |
|---|---|---|
| 133271.4 | 0.00013 | 0.97211 |

### 2.2 Determination of neutralizing activity of neutralizing antibody

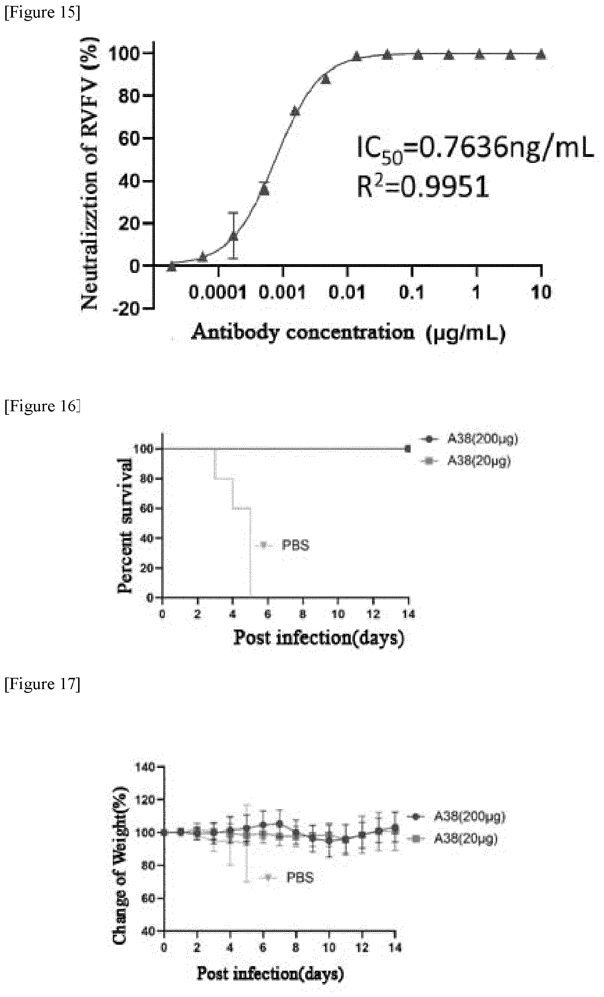

Monoclonal antibody A38 is diluted in a 96-well cell plate with ten 3-fold gradient dilutions, with 3 replicates for each dilution, added with an equal volume of eGFP-expressing RVFV (100 TCID₅₀/well) to each well, incubated at 37 °C for 60 minutes. Vero E6 cells are added to a 96-well cell plate (2 × 10⁴ cells/well), and incubated at 37 °C in a 5% CO₂ cell incubator for 48 hours. The cell supernatant is discarded, added with 4% paraformaldehyde at a dose of 300 µL/well, and fixed at room temperature for 3 hours. After washing three times with PBS, cell nuclei are stained with DAPI, and washed three times with PBS. The RVFV-infected cells (expressing eGFP) are counted by Nexcelom Celigo. The neutralizing activity of the monoclonal antibody is calculated by the formula (1-number of infected cells antibody/number of infected cells virus) × 100. Finally, the software Graphpad Prism8.0 is used to fit the neutralization curve with the four-parameter calculation method, which reveals that IC₅₀ of A38 is 0.76 ng/mL, as shown in Figure 15.

### 2.3 Efficacy evaluation of lethal mouse models

In the therapeutic activity evaluation experiment, IFNR1-/- mice aged 5-6 weeks are randomly divided into three groups: high-dose group, low-dose group, and PBS control group, with 5 mice in each group. RVFV MP-12 is diluted to 2 × 10⁵ TCID₅₀/mL, and administered intraperitoneally to mice at a dose of 100 µL for challenge. After 24 hours of challenge, A38 is administered intraperitoneally to each mouse at a dose of 200 µg in the high-dose group, and 20 µg in the low-dose group, and the control group is injected with an equal volume of PBS. After the challenge, the survival and weight changes of mice are observed every 24 hours, for 14 days. Both high and low doses of A38 can effectively protect mice with minimal weight changes, as shown in Figures 16 and 17, respectively.

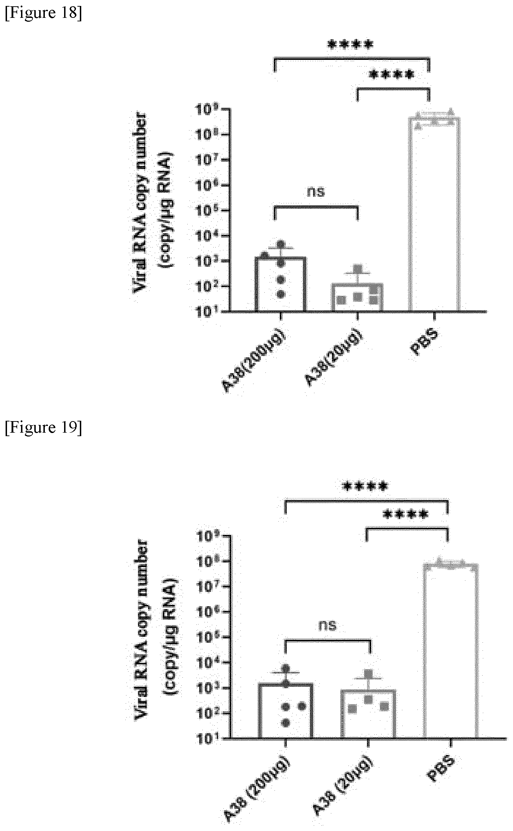

After the end of observation, the mice in the administration group are euthanized, and spleens & livers of all experimental mice are removed. The total RNA of livers and spleens is extracted by Qaigen RNeasy Mini Kit (product number: 74106), and the copy number of the viral genome in the tissue is determined by absolute fluorescence quantitative PCR. Primers and probes are shown in Table 18. Compared with the PBS control group, the copy number of the viral genome in the tissue of the administration group mice is significantly reduced, with the copy number of the viral RNA genome in livers and spleens are revealed in Figure 18 and Figure 19, respectively.

**Table 18. Fluorescence quantitative PCR primers and probe sequences**

| Name of primer | Sequence of primer |
|---|---|
| L-2912fwdGG | GAAAATTCCTGAAACACATGG |
| L-2981revAC | ACTTCCTTGCATCATCTGATG |
| L-probe-2950(5'FAM-3'BHQ1) | CAATGTAAGGGGCCTGTGTGGACTTGTG |

### 2.4 Test of antibody neutralizing mechanism

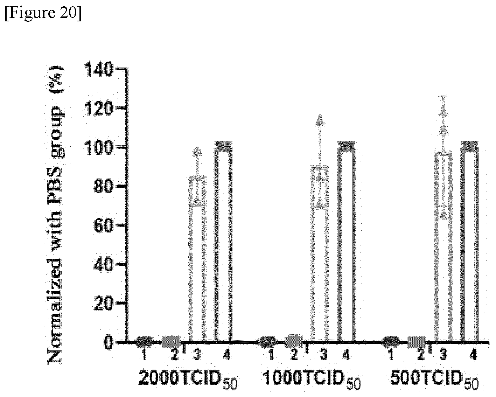

10 µg antibody is mixed with equal volumes of 2000TCID₅₀, 1000TCID₅₀, 500TCID₅₀RVFV-SeGFP, and incubated at 37 °C for 1 hour. Meanwhile, Vero E6 cells spread over a 96-well cell plate is pre-cooled at 4 °C for 15 minutes, and the supernatant is discarded. A mixture of the antibody and viruses is added to a 96-well cell plate, and incubated at 4 °C for 2 hours, to allow the virus to fully adsorb onto the cell surface. The supernatant is discarded, and cells are washed three times with pre-cooled PBS, to remove free viruses. 5% FBS DMEM medium is added, and incubated at 37 °C in a 5% CO₂ incubator for 48 hours. The supernatant is discarded, and 5% paraformaldehyde is added to fix the cells at room temperature for 3 hours, washed three times with PBS. Cell nuclei are stained with DAPI, and washed three times with PBS. The RVFV infected cells (expressing eGFP) are counted by Nexcelom Celigo. Finally, the antibody group is normalized to the PBS group. Compared with the control group of unrelated antibody 4A8 (COVID-19 neutralizing antibody), A38 can effectively block the virus adsorption to the cell surface, with comparable effect to that of the positive control antibody R15. Results are shown in Figure 20, wherein 1: A38, 2: R15, 3: A48, and 4: PBS control.

### INDUSTRIAL APPLICABILITY

The present disclosure discloses a monoclonal antibody against the Rift Valley fever virus. The antibody can be prepared industrially, showing the industrial applicability.

## Claims

1. A monoclonal antibody against RVF virus, wherein the amino acid sequence of CDR1, CDR2, and CDR3 in the heavy chain variable region of the monoclonal antibody is shown as the positions 27-35, 53-60, and 99-112 of SEQ ID NO:1 respectively; and the amino acid sequence of CDR1, CDR2, and CDR3 regions in the light chain variable region is shown as the positions 26-34, 52-58, and 97-106 of SEQ ID NO:5 respectively.

2. The monoclonal antibody of Claim 1, wherein the amino acid sequence of the heavy chain variable region of the monoclonal antibody is shown as SEQ ID NO:1, and the amino acid sequence of the light chain variable region of the monoclonal antibody is shown as SEQ ID NO: 5.

3. The antibody of Claim 2, wherein the amino acid sequence of the heavy chain constant region of the antibody is shown as SEQ ID NO:3, and the amino acid sequence of the light chain constant region of the antibody is shown as SEQ ID NO:7 or SEQ ID NO:9.

4. A polynucleotide encoding the heavy chain and light chain of the monoclonal antibody of any one of claims 1-3, wherein the sequence of the polynucleotide encoding the heavy chain variable region is shown as SEQ ID NO:2, and the sequence of the polynucleotide sequence encoding the light chain variable region is shown as SEQ ID NO:6.

5. The polynucleotide of Claim 4, wherein the sequence of the polynucleotide encoding the heavy chain constant region of the antibody is shown as SEQ ID NO:4, and the sequence of the polynucleotide encoding the light chain constant region of the antibody is shown as SEQ ID NO:8 or SEQ ID NO:10.

6. A functional element expressing the polynucleotide encoding the heavy chain and light chain of the monoclonal antibody of the claim5.

7. The functional element of Claim 6, wherein the functional element is a linear expression cassette.

8. The functional element of Claim 6, wherein the functional element is a mammalian expression vector.

9. A host cell containing the linear expression cassette of Claim 7.

10. A host cell of Claim 8, wherein the cell is an Expi 293F cell.

11. A host cell of Claim 8, wherein the cell is a CHO-S cell.

12. A use of the monoclonal antibody of any one of Claims 1-3 in the preparation of therapeutic drugs for RVF.
